# NEUE EUROPÄISCHE PATENTSCHRIFT

(11) **EP 1 538 386 B2**
(45) Veröffentlichungstag und Bekanntmachung des Hinweises auf die Entscheidung über den Einspruch: **03.11.2010**
(45) Hinweis auf die Patenterteilung: 08.08.2007
(21) Anmeldenummer: 05000973.7
(22) Anmeldetag: 31.08.2001
(51) Int. Cl.: F16M 11/04

(54) **Stativ**
Stand
Support

(30) Priorität: 30.09.2000 DE 10048545
(43) Veröffentlichungstag der Anmeldung: 08.06.2005
(62) Teilanmeldung aus: 01120922.8
(73) Patentinhaber: Carl Zeiss AG, 73447 Oberkochen (DE)
(72) Erfinder: Gaida, Gerhard, 73430 Aalen (DE); Brenner, Roland, 74599 Wallhausen (DE)

(56) Entgegenhaltungen:
- EP-A- 0 293 228
- EP-A- 0 628 290
- EP-B1- 0 048 404
- WO-A-97/13997
- WO-A1-81/03054
- DE-A- 10 142 564
- DE-A- 19 820 710
- DE-A1- 4 320 443
- DE-A1- 19 742 050
- DE-B2- 2 320 266
- DE-U- 20 019 105
- DE-U- 20 114 343
- DE-U1- 20 019 107
- JP-A- 04 013 591
- US-A- 3 396 931
- US-A- 3 945 597
- US-A- 4 344 595
- US-A- 4 364 535
- US-A- 4 548 373
- US-A- 4 741 607
- US-A- 5 494 034
- W.H.LANG F.MUCHEL ZEISS MICROSCOPES FOR MICROSURGERY Januar 1981, SPRINGER-BVERLAG BERLIN, Seiten 64 - 73
- ERNST LEITZ: 'Vorbildlicher Unternehmer und mutiger Demokrat' WEGBEREITER DER LEICA 01 Januar 2006, KÖNIGSWINTER, ISSN 10389880 Seiten 55 - 61

## Beschreibung

Die Erfindung betrifft ein Stativ, insbesondere zur Aufnahme eines medizinischen Gerätes, beispielsweise eines Operationsmikroskops, mit einer Ständersäule, an der ein erster Trägerarm angeordnet ist und mit einem zweiten Trägerarm, der an dem ersten Trägerarm aufgenommen ist und relativ zu dem ersten Trägerarm um eine erste Achse und um eine von der ersten Achse verschiedenen zweiten Achse bewegt werden kann, bei dem zum Erzeugen einer Rückstellkraft für den zweiten Trägerarm um die erste Achse ein bewegbares Gewicht vorgesehen ist, und Mittel zum Koppeln vorgesehen sind, welche das Gewicht mit einer Bewegung des zweiten Trägerarmes um die erste Achse koppeln.

Ein derartiges Stativ ist aus der US 4,548,373 bekannt. Dort ist ein Stativ für ein Operationsmikroskop beschrieben, welches eine erste Ständersäule hat, an der um eine vertikale Drehachse ein erster Trägerarm schwenkbeweglich aufgenommen ist. Dieser erste Trägerarm trägt mit einem Endabschnitt einen zweiten Trägerarm. Dieser zweite Trägerarm kann zu dem ersten Trägerarm in einer Gelenkaufnahme um eine erste horizontal ausgerichtete Drehachse und um eine zu der ersten horizontal ausgerichteten Drehachse senkrecht ausgerichteten weiteren Drehachse bewegt werden. An dem zweiten Trägerarm befindet sich ein Ausgleichsgewicht, das zum Ausgleich des an dem zweiten Trägerarm aufgenommenen Lastgewichts des Operationsmikroskops dient.

Aus der DE 198 20 710 A1 ist ein Stativ zur Aufnahme einer Beleuchtungseinheit bekannt. Dieses Stativ umfasst eine Ständersäule, an der ein Trägerarm aufgenommen ist. Dieser Trägerarm kann bezüglich der Ständersäule in einer horizontalen Ebene gedreht werden und in einer vertikalen Ebene zur Ständersäule verschwenkt werden. Zum Ausgleich eines durch eine am Trägerarm aufgenommene Last hervorgerufenen Last-Drehmoments ist an der Ständersäule ein bewegbar angeordnetes Gewicht vorgesehen, das mit dem Trägerarm in Wirkverbindung steht.

Die US 5,494,034 offenbart ein Bodenstativ, welches eine Ständersäule umfaßt, an der eine Trägervorrichtung mit einem ersten und einem zweiten Trägerarm angeordnet ist. Der erste Trägerarm ist an der Ständersäule drehbar gelagert. An einem Endbereich des ersten Trägerarmes ist der zweite Trägerarm schwenkbeweglich befestigt und bzgl. zwei zu dem ersten Trägerarm orthogonalen Achsen bewegbar. Ein dritter Trägerarm ist wiederum an einem Ende des zweiten Trägerarmes drehbar angeordnet und hält mit einem Drehgelenk ein chirurgisches Instrument. In der US 5,494,034 wird vorgeschlagen, die Gelenkverbindung von zweitem und drittem Trägerarm und das Drehgelenk, an dem das chirurgische Instrument angeordnet ist, mit Zahnriemengetrieben zu kombinieren, welche die Bewegung des dritten Trägerarmes und des chirurgischen Instruments an einen Gewichtsausgleichsmechanismus koppeln. Dieser Gewichtausgleichsmechanismus umfasst zwei Ausgleichgewichte, die an Hebelarmen aufgenommen sind und mit einer gabelförmig ausgebildeten Halteeinrichtung von dem ersten Trägerarm im Bereich einer Gelenkverbindung vom ersten und zweiten Trägerarm getragen werden.

In der EP 0 293 228 B 1 ist ein Stativ für ein Operationsmikroskop beschrieben, bei dem ein Gestängemechanismus zur Kopplung von Operationsmikroskop und Gegengewichten vorgesehen ist. Mittels dieser Gegengewichte ist es möglich, das Stativ auszubalancieren und somit das Operationsmikroskop nahezu frei von Gewichtskräften zu bewegen.

Aus der WO 97/13997 ist ein Bodenstativ mit Gewichtsausgleich zur Aufnahme eines Operationsmikroskops bekannt. Dieses Stativ hat eine Ständersäule, an der eine Trägerarmvorrichtung mit einem ersten und einem zweiten Trägerarm aufgenommen ist. Dabei ist der erste Trägerarm an der Ständersäule schwenkbeweglich befestigt. Der zweite Trägerarm ist wiederum am ersten Trägerarm drehbar gelagert. Das Operationsmikroskop ist in einem Endbereich des zweiten Trägerarmes aufgenommen. Zum Ausgleich der am Operationsmikroskop angreifenden Gewichtskraft sind am ersten und zweiten Trägerarm jeweils Gegengewichte vorgesehen. Diese Gegengewichte balancieren den ersten und zweiten Trägerarm nach dem Prinzip einer Balkenwaage aus.

Aus der DE 200 19 105 U1 ist ein Stativ bekannt, das eine Ständersäule aufweist, aus der ein erster Trägerarm um eine Drehachse der Ständersäule drehbeweglich gelagert ist. Ein zweiter Trägerarm ist an dem ersten Trägerarm aufgenommen und relativ zu diesem um eine erste Achse und eine zweite, orthogonal zur ersten Achse und parallel zu Drehachse der Ständersäule ausgerichteten Achse bewegbar. In der Ständersäule ist ein Gewicht angeordnet, das über einen Seilzug mit einem an dem ersten Trägerarm angeordneten Tragarm verbunden ist, wobei der Seilzug über ein Tariergetriebe mit dem Tragarm gekoppelt ist. Über das Gewicht wird eine an dem Tragarm aufgenommene Last kompensiert.

Aufgabe der Erfindung ist es, ein Stativ bereitzustellen, das einerseits platzsparende Abmessungen aufweist, andererseits jedoch ermöglicht, daß ein schweres, am Stativ aufgenommenes medizinisches Gerät leicht in beliebige Richtungen bewegt werden kann.

Diese Aufgabe wird durch ein Stativ mit den Merkmalen des Anspruchs 1 gelöst.

Bei einem Stativ gemäß Anspruch 1 ist das Gewicht im Bereich einer Ständersäule bewegbar angeordnet. Unter einem Gewicht, das im Bereich einer Ständersäule bewegbar angeordnet ist, wird dabei ein Gewicht verstanden, welches sich nahe bei bzw. in der Ständersäule befindet und dessen Position dort verändert werden kann. Indem es auf oder unmittelbar bei einer Drehachse der Ständersäule angeordnet ist, können Trägheitskräfte minimiert werden, die bei Drehung des Stativs um die Drehachse der Ständersäule auftreten. Diese Trägheitskräfte haben ihre Ursache in dem Gewicht und wirken der betreffenden Stativbewegung um die entsprechende Drehachse mit einem Gegenmoment entgegen. Eine solche Anordnung des Gewichts gewährleistet also eine leichte Drehbarkeit des Stativs um eine Achse der Ständersäule. Außerdem kann so ein kippstabiles Stativ mit niedrigem Geräteschwerpunkt geschaffen werden.

Das Gewicht bei dem erfindungsgemäßen Stativ wirkt als Gegengewicht. Auf diese Weise kann unter Beibehaltung einer einfachen Konstruktion durch Vergrößern oder Verkleinern des Gegengewichts eine Rückstellkraft für ein unterschiedliches Gewicht des von dem Stativ aufgenommenen medizinischen Geräts eingestellt werden.

In Weiterbildung der Erfindung gleichen die Mittel zum Erzeugen einer Rückstellkraft eine an dem zweiten Arm angreifende Gewichtskraft aufgrund einer daran angeordneten Last aus. Auf diese Weise ist es möglich, das Stativ vollständig auszubalancieren, so daß das am Stativ aufgenommene medizinische Gerät nahezu kraftlos bewegt werden kann.

In Weiterbildung der Erfindung wird bei dem Stativ über den Seilzug eine ausgleichende Rückstellkraft übertragen, die an dem zweiten Arm in einem Abstand von der Ständersäule angreift, wobei der Seilzug in eine Parallelrichtung zur Ständersäule umgelenkt ist. Auf diese Weise kann ein Gegengewicht im Bereich der Ständersäule angeordnet werden, um so ein kippstabiles Stativ bereitzustellen, dessen Schwerpunkt im Bereich der Ständersäule liegt.

Für den Seilzug kann eine Umlenkung zum Führen des Seilzugs in der Nähe einer Ständersäule vorgesehen werden. Auf diese Weise ist es möglich, in der Ständersäule ein Ausgleichsgewicht zu führen.

In Weiterbildung der Erfindung ist beim Stativ der erste Trägerarm um eine zur Ständersäule im wesentlichen parallele Drehachse drehbar. Auf diese Weise wird ein Stativ mit großem Arbeitsbereich bereitgestellt.

Die Mittel zum Koppeln können wenigstens einmal unterbrochen sein. Auf diese Weise wird eine leichte Bewegbarkeit des Stativs gewährleistet. Insbesondere bei Verwendung eines Seilzuges als Mittel zum Koppeln oder einer Gestängeanordnung wird ein Verdrillen oder Verwinden von Seilzug bzw. Gestänge vermieden. Vorzugsweise ist den Mitteln zum Koppeln ein als Drehentkopplung wirkendes Freilaufdrehgelenk zugeordnet, welches ein Rückkoppeln einer Bewegung des zweiten Arms um wenigstens eine der beiden Achsen zu dem Mittel zum Erzeugen einer Rückstellkraft vermeidet. Auf diese Weise wird ein Stativ geschaffen, bei dem ein Rückstellmoment für eine Achse zum Ausgleich einer entsprechenden Stativbelastung keinerlei Auswirkungen auf die Bewegbarkeit des Stativs um eine andere Achse hervorruft.

Ein Freilaufdrehgelenk ermöglicht insbesondere das Verdrillen eines Seilzugs als Mittel zum Koppeln zu unterbinden.

Unter einer im wesentlichen orthogonalen Orientierung von erster und zweiter Achse wird eine Orientierung dieser Achsen verstanden, bei der diese innerhalb eines Toleranzintervalls um ± 20° senkrecht aufeinander stehen. Auf diese Weise wird ein Trägerarmsystem mit zwei Bewegungsfreiheitsgraden bereitgestellt, deren Gang von einem Benutzer des Stativs leicht erfaßt werden kann.

In Weiterbildung der Erfindung sind die erste und die zweite Achse in Abstand voneinander angeordnet. Auf diese Weise wird eine mechanisch einfache Bauform der Stativgelenke ermöglicht.

In Weiterbildung der Erfindung liegt das Freilaufdrehgelenk auf einer der Achsen. Auf diese Weise kann als Freilaufdrehgelenk ein Wirbelblock verwendet werden, der die nahezu vollständige Unterdrückung einer Rückkopplung bewirkt.

In Weiterbildung der Erfindung umfaßt das Stativ als Mittel zum Erzeugen einer Rückstellkraft einen Hebelarm, der an einem der Trägerarme angeordnet ist und ein Gegengewicht trägt, wobei die Mittel zum Koppeln eine Schwenkbewegung des Hebelarms an eine Schwenkbewegung des zweiten Trägerarms koppeln. Auf diese Weise ist es möglich. ein mit sich ändernder Stellung des medizinischen Geräts variierendes Lastmoment durch ein an das Lastmoment angepaßte Gegenmoment auszugleichen.

In Weiterbildung der Erfindung ist das Stativ als Bodenstativ ausgebildet. Auf diese Weise wird ein leicht transportables Stativ bereitgestellt.

Wird das Stativ mit dieser Bauform als Deckenstativ ausgebildet, so wird ein besonders platzsparender Stativaufbau geschaffen.

Weitere Merkmale und Vorteile der Erfindung sind in den Zeichnungen dargestellt und werden nachfolgend beschrieben.

Es zeigen:
- Figur 1:: eine erste Ausführungsform eines Stativs;
- Figur 2:: den Aufbau eines Freilaufdrehgelenks in einem Stativ aus Figur 1; und

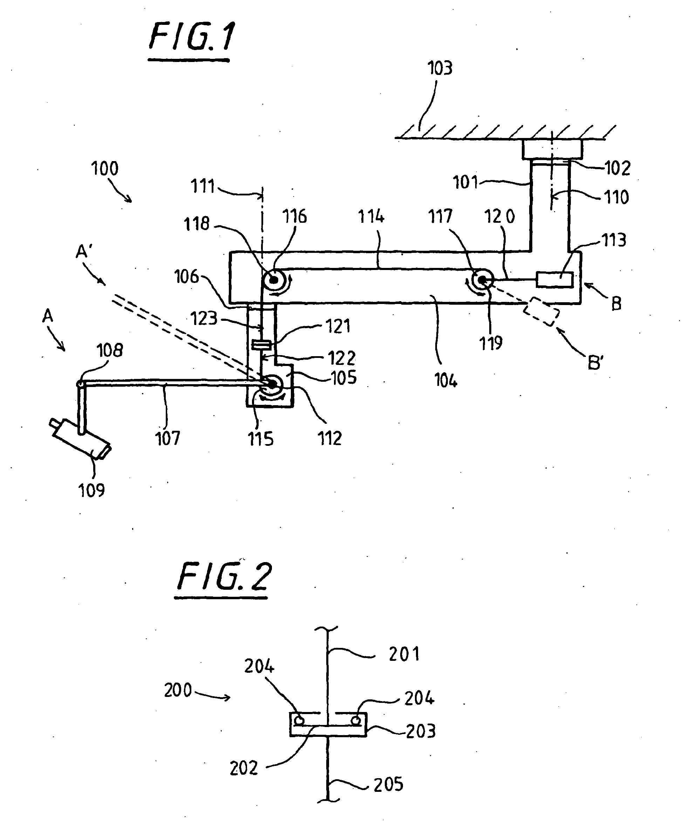

Die Figur 1 zeigt ein als Deckenstativ ausgebildetes Stativ 100, welches mittels einer Ständersäule 101 mit Drehgelenk 102 an einer Decke 103, beispielsweise in einem Operationssaal aufgehängt ist. In Alternative zur Ausführung des Stativs 100 als Deckenstativ ist es auch möglich, unter Beibehaltung des wesentlichen Konstruktionsprinzips, dieses als Bodenstativ auszubilden. In diesem Fall wird beispielsweise die Ständersäule 101 mit Drehgelenk 102 auf einen geeigneten Stativfuß montiert.

Die Ständersäule 101 ist mit einem als Tragarm wirkenden ersten Trägerarm 104 verbunden, an dem in einem Endbereich über einen Hubarmträger 105 mit Drehgelenk 106 ein zweiter Trägerarm 107 aufgenommen ist. Dieser zweite Trägerarm 107 wirkt als Hubarm.

Er hat in einem Endbereich ein Gelenk 108, an dem ein Operationsmikroskop 109 als medizinisches Gerät gelagert ist.

Der Trägerarm 104 kann aufgrund des Drehgelenks 102 an der Ständersäule 101 um eine zur Ständersäule parallele Drehachse 110 geschwenkt werden. Das Drehgelenk 106 im Hubarmträger 105 gewährleistet, dass der zweite Trägerarm 107 um eine Drehachse 111 schwenkbar ist. Die Drehachse 111 verläuft dabei im wesentlichen parallel zur Ständersäule 101 und liegt beim anhand von Figur 1 erläuterten Ausführungsbeispiel in der Zeichenebene. Unter einem im wesentlichen parallelen Verlauf von Drehachse 111 und Ständersäule 101 wird dabei auch ein Verlauf verstanden, der beispielsweise bedingt durch Fertigungstoleranzen von einem strengen parallelen Verlauf abweicht.

Am Hubarmträger 105 ist der zweite Trägerarm 107 in Abstand vom ersten Trägerarm 104 an einer Schwenkachse 112 schwenkbeweglich gelagert. Diese Schwenkachse verläuft einerseits orthogonal zur Drehachse 111 des Hubarmträgers 105 und ist in der Figur senkrecht zur Zeichenebene orientiert. Diese Aufhängung des Hubarmträgers 105 ermöglicht es, beispielsweise den Trägerarm 107 aus einer Schwenkposition A in eine mittels gestrichelten Linien angedeutete Schwenkposition A' zu bewegen.

Um eine an dem Operationsmikroskop 109 angreifende Gewichtskraft auszugleichen, die an der Schwenkachse 112 einen Moment hervorruft, welches bestrebt ist, das Operationsmikroskop 109 abzusenken, ist bei dem Stativ 100 ein Gegengewicht 113 vorgesehen. Dieses Gegengewicht 113 dient als Mittel zum Erzeugen einer Rückstellkraft. Das Gegengewicht 113 ist im Bereich der Ständersäule 101 angeordnet. Damit wird eine Trägheitswirkung des Gegengewichts 113 bei einer Bewegung der Ständersäule 101 mit den Trägerarmen 104 und 107 um die Drehachse 110 minimiert, bei der das Gegengewicht ein unerwünschtes Gegenmoment hervorruft und somit die Bewegung um die Drehachse 110 erschweren würde. Ferner liegt so der Gesamtschwerpunkt des Stativs bei aufgenommenem Operationsmikroskop im Bereich der Ständersäule 101 in der Nähe der Drehachse 110 und es werden beim Drehgelenk 102 solche Lagerkräfte minimiert, die senkrecht zu dessen Drehachse 110 orientiert sind.

Das Gegengewicht 113 ist über einen Seilzug 114 als Mittel zum Koppeln mit einer Umlenkrolle 115 verbunden, über die ein Moment auf die Schwenkachse 112 aufgebracht wird, das die Gewichtskraft des Operationsmikroskops 109 ausgleicht. Dieser Seilzug 114 koppelt somit das Gegengewicht 113 mit einer Bewegung des zweiten Trägerarms 107 um die Schwenkachse 112. Der Seilzug 114 wird von der Umlenkrolle 115 entlang der Drehachse 111 des Drehgelenks 106 in dem Hubarmträger 105 geführt. Mittels einer Umlenkrolle 116 wird es zum ersten Trägerarm 104 hin umgelenkt und auf eine Umlenkrolle 117 gelegt, an der das von dem Gegengewicht 113 hervorgerufene Gegenmoment angreift. Bei dem in der Figur 1 gezeigten Ausführungsbeispiel sind die Umlenkrollen 116 und 117 auf Drehachsen 118, 119 gelagert, die jeweils zum ersten Trägerarm 104 und zur Zeichenebene senkrecht orientiert sind. Das Gegengewicht 113 greift an der Umlenkrolle 117 mit einem Hebelarm 120 an. Bei Bewegung des zweiten Trägerarmes 107 mit einem an ihm aufgenommenen Operationsmikroskop 109 aus einer ersten Schwenkposition A in die zweite mit gestrichelten Linien angedeutete Schwenkposition A' wird das an dem Hebelarm 120 aufgenommene Gegengewicht 113 aus der Stellung B in die Stellung B' bewegt, die in entsprechender Weise mit gestrichelten Linien angedeutet ist. In den jeweiligen Stellungen des Gegengewichts 113 sind die resultierenden Hebelarme unterschiedlich und an die momentane Stellung des zweiten Trägerarmes 108 angepasst. Dies gewährleistet, dass trotz eines sich ändernden Lastmoments, das an der Schwenkachse 112 angreift, wenn der zweite Trägerarm 107 aus der Position A in die Position A' bewegt wird, mittels des Gegengewichts 113 eine gerade benötigte Ausgleichskraft erzeugt wird.

Im Bereich des Hubarmträgers 105 ist im Seilzug 114 auf der Drehachse 111 ein Freilaufdrehgelenk 121 vorgesehen. Dieses Freilaufdrehgelenk 121 bewirkt, dass sich bei einer Bewegung des zweiten Trägerarmes 107 um die Drehachse 111 das Seil des Seilzuges 114 nicht verdrillt. Außerdem unterteilt das Freilaufdrehgelenk 121 den Seilzug 114 in einen dem zweiten Trägerarm 107 zugeordneten Abschnitt 122 und einen Abschnitt 123, der zum Gegengewicht 113 weist. Diese betreffenden Abschnitte 122 und 123 des Seilzugs 114 sind somit zueinander frei drehbar, so dass eine Drehbewegung des zweiten Trägerarmes 107 um die Drehachse 111 von einer Bewegung des Gegengewichts 113 völlig entkoppelt ist.

Insbesondere führt eine Drehbewegung des zweiten Trägerarmes 107 um die Drehachse 111 nicht zu einer Rückkopplung dieser Drehbewegung in den Seilzugabschnitt 123, an dem das Gegengewicht 113 angeordnet ist. Außerdem wird bei dieser Anordnung des Gegengewichts 113 die Trägheit des Gegengewichts 113 bei einer Drehung des zweiten Trägerarmes 107 um die Drehachse 111 des Drehgelenks 106 am Hubarmträger minimiert.

Eine mögliche Ausführungsform für das Freilaufdrehgelenk 120 aus Figur 1 ist in der Figur 2 gezeigt. Dieses Freilaufdrehgelenk ist als kugelgelagertes Drehgelenk 200 ausgebildet. Der zum Gegengewicht 113 aus Figur 1 weisende Abschnitt des Seilzuges 201 ist mit einer Kugelträgerplatte 202 verbunden. Der zum Hubarm 107 aus Figur 1 weisende Abschnitt des Seilzuges 205 ist wiederum an einem Kugellagergehäuse 203 festgelegt. Zwischen der Kugelträgerplatte 202 und dem Kugellagergehäuse 203 sind Kugeln 204 angeordnet. Dieser Aufbau ermöglicht, dass sich bei einer Drehung des zweiten Trägerarmes 107 aus Figur 1 um die Drehachse 111 aus Figur 1 das Kugellagergehäuse 203 zusammen mit dem zum zweiten Trägerarm weisenden Abschnitt des Seilzuges 205 frei drehen kann, ohne den zum Gegengewicht 113 weisenden Abschnitt des Seilzuges 201 nennenswert zu verdrillen.

Anstatt das Freilaufdrehgelenk wie anhand der Figur 2 erläutert als kugelgelagertes Drehgelenk auszubilden, ist es auch möglich, dieses als Wirbelblock oder in einer anderen bekannten Weise auszuführen.

## Patentansprüche

1. Stativ (100), insbesondere zur Aufnahme eines medizinischen Geräts, beispielsweise eines Operationsmikroskops (109),
- mit einer Ständersäule (101), an der ein erster Trägerarm (104) angeordnet ist, wobei der erste Trägerarm (104) um eine Drehachse (110 ) der Ständersäule (101) gedreht werden kann, und
- mit einem zweiten Trägerarm (107), der an dem ersten Trägerarm aufgenommen ist und relativ zu dem ersten Trägerarm (104) um eine erste Achse (112) und um eine von der ersten Achse verschiedenen zweiten Achse (111) bewegt werden kann, wobei die erste Achse (112) im Wesentlichen orthogonal zu der zweiten Achse (111) angeordnet ist und wobei die zweite Achse (111) parallel zur Drehachse (110) der Ständersäule (101) verläuft,
- bei dem zum Erzeugen einer Rückstellkraft für den zweiten Trägerarm (107 ) um die erste Achse (112) ein bewegbares Gewicht (113) vorgesehen ist, und
- Mittel zum Koppeln (114) vorgesehen sind, welche das Gewicht (113) mit einer Bewegung des zweiten Trägerarmes (107) um die erste Achse (112) koppeln, um die
wobei das bewegbare Gewicht (113) im Bereich der Ständersäule (101) angeordnet ist und im Bereich der Ständersäule (101) bewegbar ist und zur Kopplung von Gewicht (113) und zweitem Trägerarm (107) ein Seilzug (114) vorgesehen ist, **dadurch gekennzeichnet, dass** eine Umlenkrolle (115) vorgesehen ist, wobei das bewegbare Gewicht (113) über den Seilzug (114) mit der Umlenkrolle verbunden ist und wobei über die Umlenkrolle (115) ein Moment um die erste Achse (112) aufbringbar ist, und wobei der Seilzug (114) von der Umlenkrolle (115) entlang der zweiten Achse (111) geführt ist.

2. Stativ gemäß Anspruch 1, **dadurch gekennzeichnet, dass** das Gewicht (113) eine an dem zweiten Arm (107) angreifende Gewichtskraft aufgrund einer daran angeordneten Last (109) ausgleicht.

3. Stativ gemäß einem der Ansprüche 1 bis 2, **dadurch gekennzeichnet, dass** der erste Trägerarm (104) um eine zu der Ständersäule (101) im Wesentlichen parallele Drehachse (110) drehbar ist.

4. Stativ gemäß einem der Ansprüche 1 bis 3, **dadurch gekennzeichnet, dass** die Mittel zum Koppeln (114) zum Unterdrücken einer Rückkopplung einer Bewegung des zweiten Trägerarms (107) um die zweite Achse (111) zu dem Gewicht ein als Drehentkopplung wirkendes Freilaufdrehgelenk (121) aufweisen.

5. Stativ gemäß Anspruch 4, **dadurch gekennzeichnet, dass** das Freilaufdrehgelenk (121) auf einer der Achsen (111) liegt.

6. Stativ gemäß einer der Ansprüche 4 oder 5, **dadurch gekennzeichnet, dass** das Freilaufdrehgelenk (121) als kugelgelagertes Drehgelenk (200) oder als Wirbelblock ausgeführt ist.

7. Stativ gemäß einem der Ansprüche 1 bis 6, **dadurch gekennzeichnet, dass** die erste Achse (112) und die zweite Achse (111) in Abstand voneinander angeordnet sind.

8. Stativ gemäß einem der Ansprüche 1 bis 7, **dadurch gekennzeichnet, dass** dem bewegbaren Gewicht (113) ein Hebelarm (120) zugeordnet ist, der an einem der Trägerarme (104) angeordnet ist und das Gewicht (113) trägt, wobei die Mittel zum Koppeln (114) eine Schwenkbewegung des Hebelarmes (120) an eine Schwenkbewegung des zweiten Trägerarmes (107) koppeln.

9. Stativ gemäß einem der Ansprüche 1 bis 8, **dadurch gekennzeichnet, dass** das Stativ als Bodenstativ ausgebildet ist.

10. Stativ gemäß einem der Ansprüche 1 bis 8, **dadurch gekennzeichnet, dass** das Stativ als Deckenstativ (100) ausgebildet ist.

## Claims

1. Support (100), in particular for receiving a medical appliance, for example an operation microscope (109),
- with a support column (101), on which a first carrier arm (104) is arranged, it being possible for the first carrier arm (104, 304) to be turned about a rotation axis (110) of the support column (101), and
- with a second carrier arm (107) which is mounted on the first carrier arm and can be moved relative to said first carrier arm (104) about a first axis (112) and about a second axis (111) different from the first axis, the first axis (112) being arranged substantially orthogonal to the second axis (111) and the second axis (111) running parallel to the rotation axis (110) of the support column (101),
- a movable weight (113) being provided for producing a restoring force for the second carrier arm (107) about the first axis (112), and
- coupling means (114) being provided which couple the weight (113) to a movement of the second carrier arm (107) about the first axis (112),
the movable weight (113) being arranged in the area of the support column (101) and being movable in the area of the support column (101) and a cable (114) being provided for coupling the weight (113) and the second carrier arm (107), **characterized in that** a deflecting roller (115) is provided, the movable weight (113) being connected to the deflecting roller via the cable (114) and it being possible for a moment to be applied about the first axis (112) by means of the deflecting roller (115), and the cable (114) being guided by the deflecting roller (115) along the second axis (111).

2. Support according to Claim 1, **characterized in that** the weight (113) compensates for a weight force impinging on the second arm (107) because of a load (109) arranged on it.

3. Support according to either of Claims 1 and 2, **characterized in that** the first carrier arm (104) can turn about a rotation axis (110) substantially parallel to the support column (101).

4. Support according to one of Claims 1 to 3, **characterized in that** the coupling means (114), in order to suppress a feedback coupling of a movement of the second carrier arm (107) about the second axis (111) to the weight, have a freewheel rotary joint (121) acting as rotation decoupler.

5. Support according to Claim 4, **characterized in that** the freewheel rotary joint (121) lies on one of the axes (111).

6. Support according to either of Claims 4 and 5, **characterized in that** the freewheel rotary joint (121) is designed as a ball-bearing rotary joint (200) or as a swivel block.

7. Support according to one of Claims 1 to 6, **characterized in that** the first axis (112) and the second axis (111) are arranged at a distance from one another.

8. Support according to one of Claims 1 to 7, **characterized in that** the movable weight (113) is assigned a lever arm (120) which is arranged on one of the carrier arms (104) and bears the weight (113), and the coupling means (114) couple a pivoting movement of the lever arm (120) to a pivoting movement of the second carrier arm (107).

9. Support according to one of Claims 1 to 8, **characterized in that** the support is designed as a floor support stand.

10. Support according to one of Claims 1 to 8, **characterized in that** the support is designed as a ceiling support (100).

## Revendications

1. Support (100), en particulier pour recevoir un appareil médical, par exemple un microscope d'opération (109),
- comprenant une colonne de support (101) sur laquelle est disposé un premier bras de support (104), le premier bras de support (104, 304) pouvant être tourné autour d'un axe de rotation (110) de la colonne de support (101), et
- un deuxième bras de support (107), qui est reçu sur le premier bras de support et qui peut être déplacé par rapport au premier bras de support (104) autour d'un premier axe (112) et autour d'un deuxième axe (111) différent du premier axe, étant essentiellement perpendiculaire au deuxième axe (111), et le deuxième axe (111) s'étendant parallèlement à l'axe de rotation (110) de la colonne de support (101),
- un poids mobile (113) étant prévu pour produire une force de rappel pour le deuxième bras de support (107) autour du premier axe (112), et
- des moyens d'accouplement (114) étant prévus pour accoupler le poids (113) à un mouvement du deuxième bras de support (107) autour du premier axe (112),
le poids mobile (113) étant disposé dans la région de la colonne de support (101) et pouvant être déplacé dans la région de la colonne de support (101), et
pour l'accouplement du poids (113) et du deuxième bras de support (107), un câble de traction (114) étant prévu, **caractérisé en ce qu'**une poulie de renvoi (115) est prévue, le poids mobile (113) étant connecté à la poulie de renvoi par le biais du câble de traction (114) et un couple pouvant être appliqué autour du premier axe (112) par le biais de la poulie de renvoi (115), et
le câble de traction (114) étant guidé par la poulie de renvoi (115) le long du deuxième axe (111).

2. Support selon la revendication 1, **caractérisé en ce que** le poids (113) compense une force de pesanteur appliquée sur le deuxième bras (107), du fait d'une charge (109) disposée sur celui-ci.

3. Support selon l'une quelconque des revendications 1 ou 2, **caractérisé en ce que** le premier bras de support (104) peut tourner autour d'un axe de rotation (110) substantiellement parallèle à la colonne de support (101).

4. Support selon l'une quelconque des revendications 1 à 3, **caractérisé en ce que** les moyens d'accouplement (114) présentent une articulation pivotante de roue libre (121) agissant en tant que désaccouplement de rotation pour supprimer une rétroaction d'un mouvement du deuxième bras de support (107) autour du deuxième axe (111) vers le poids.

5. Support selon la revendication 4, **caractérisé en ce que** l'articulation pivotante de roue libre (121) repose sur l'un des axes (111).

6. Support selon l'une quelconque des revendications 4 ou 5, **caractérisé en ce que** l'articulation pivotante de roue libre (121) est réalisée sous la forme d'une articulation pivotante à roulement (200) ou sous la forme d'un bloc rotationnel.

7. Support selon l'une quelconque des revendications 1 à 6, **caractérisé en ce que** le premier axe (112) et le deuxième axe (111) sont espacés l'un de l'autre.

8. Support selon l'une quelconque des revendications 1 à 7, **caractérisé en ce que** l'on associe au poids mobile (113) un bras de levier (120) qui est disposé sur l'un des bras de support (104) et qui porte le poids (113), les moyens d'accouplement (114) couplant un mouvement de pivotement du bras de levier (120) à un mouvement de pivotement du deuxième bras de support (107).

9. Support selon l'une quelconque des revendications 1 à 8, **caractérisé en ce que** le support est réalisé sous la forme d'un support au sol.

10. Support selon l'une quelconque des revendications 1 à 8, **caractérisé en ce que** le support est réalisé sous la forme d'un support au plafond (100).
